# DEMANDE DE BREVET EUROPEEN

(11) **EP 0 834 504 A1**
(43) Date de publication de la demande: **08.04.1998**
(21) Numéro de dépôt: 97402313.7
(22) Date de dépôt: 02.10.1997
(51) Int. Cl.: C07D 213/22, C07D 213/57, C07D 213/30, G02F 1/35

(54) **Composés organiques conjugués, procédé de préparation, matériaux en dérivant, utilisation dans des dispositifs électroniques, opto-électroniques, optiques non linéaires et électro-optiques**

(30) Priorité: 03.10.1996 FR 9612066
(71) Demandeur: Office National d'Etudes et de Recherches Aerospatiales "ONERA", 92322 Chatillon (FR)
(72) Inventeur: Attias, André-Jean, 75015 Paris (FR); Bloch, Bertrand, 75005 Paris (FR); Cavalli, Chantal, 78960 Voisins le Bretonneux (FR)
(74) Mandataire: Warcoin, Jacques

(57) **Abrégé**

L'invention concerne des composés conjugués de formule générale (I) 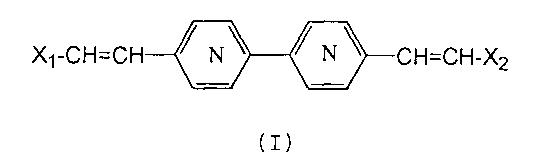 dans laquelle
X₁ et X₂ sont indépendamment l'un de l'autre des systèmes électroaccepteurs ou électrodonneurs, ainsi qu'un procédé de préparation de ces composés.

L'invention concerne également tout matériau incluant les composés de formule générale I et l'utilisation desdits composés ou de tout matériau les incluant dans des dispositifs électroniques, opto-électroniques, optiques-non-linéaires et électro-optiques.

## Description

La présente invention concerne de nouveaux composés organiques conjugués de formule générale I : 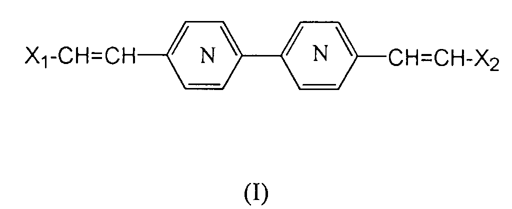 dans laquelle
X₁ et X₂ sont indépendamment l'un de l'autre des systèmes conjugués aliphatiques eux-mêmes conjugués avec le reste de la molécule, cycliques aromatiques ou hétérocycliques aromatiques, en particulier un phényle ou un thiényle, non substitués ou substitués une ou plusieurs fois par des radicaux choisis parmi le groupe alkyle, halogéno, phényle, naphtyle, hydroxy, alkoxy, amino, alkyl inférieur-amino, dialkyl inférieur-amino, alkyl inférieur-alkoxy inférieur-amino, (alkyl inférieur-acyl)-amino-carbonyle, alkyl inférieur-carbonyle, alkoxy inférieur-carbonyle, carboxyle, (alkyl inférieur-amino)-carbonyle, (dialkyl inférieur-amino)-carbonyle, halogéno-carbonyle, trifluoro-méthyle, nitro, nitroso, cyano, 2,2-dicyano-vinyle, 3,3-dicyano-prop-2-ényle,tricyano-vinyle, dicyanométhylidényle, mercapto, alkylthio, sulfino, alkyl-sulfonyle, sulfo, alkyl-sulfonate, alkyl-sulfinyle, pyridinyle et dérivés de pyridinyle conjugués.

On entend par alkyle inférieur, les radicaux alkyle linéaires ou ramifiés comprenant 1 à 6 atomes de carbone.

L'entité 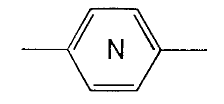 représente un radical hétérocyclyle aromatique azoté comprenant 1 à 4 atomes d'azote choisi parmi pyridinyle, pyrazinyle, pyrimidinyle, triazinyle, et comportant au moins un atome d'azote en position α par rapport à la double liaison CH=CH.

En particulier, la présente invention comprend les composés de formule générale I dans laquelle l'entité 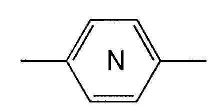 représente un radical pyridinyle comportant un atome d'azote en position α par rapport à la double liaison CH=CH, et dans laquelle X₁ et X₂ représentent indépendamment l'un de l'autre de préférence un phényle, un 4-cyano-phényle ou un 4-hydroxy-phényle.

La présente invention concerne la préparation du composé de formule générale I, en particulier la [(6-X₁-CH=CH)-(6'-X₂-CH=CH)]-3,3'-bipyridine. Suivant la nature des substituants X₁ et X₂, les conditions opératoires du procédé de préparation selon l'invention du composé de formule générale I varient.

Si X₁ = X₂, on condense le produit de formule générale II 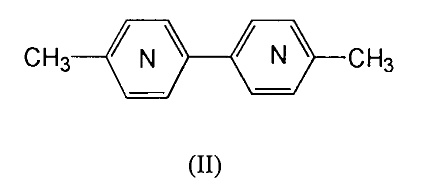 dans laquelle l'entité 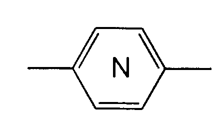 représente un radical hétérocyclyle aromatique azoté comprenant 1 à 4 atomes d'azote choisi parmi pyridinyle, pyrazinyle, pyrimidinyle, triazinyle, et comportant au moins un atome d'azote en position α par rapport au radical méthyle, avec un excès d'aldéhyde de formule X-CHO avec X=X₁=X₂, X₁ et X₂ étant définis comme précédemment.

Par contre si X₁ ≠ X₂, on condense le produit de formule générale II, dans laquelle l'entité 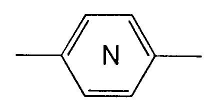 a la même signification que précédemment, successivement par un équivalent molaire d'aldéhyde de formule X₁-CHO, dans laquelle X₁ est défini ci-dessus, puis par un équivalent molaire d'aldéhyde X₂-CHO, dans laquelle X₂ ≠ X₁ et X₂ est défini ci-dessus. La réaction peut être conduite en présence d'un catalyseur et, éventuellement, d'un déshydratant. Un même composé, tel l'anhydride benzoïque, peut réaliser les deux fonctions précitées.

Cette réaction de condensation, connue sous le nom de réaction de Knoevenagel est décrite dans Ber. 31,2596(1898) et Journal of Polymer Science, A1(7), 743:752(1969). Elle est fortement influencée par la nature des substituants X₁ et X₂ des aldéhydes utilisés. Un substituant électro-accepteur favorisera la réaction tandis qu'un substituant électrodonneur en diminuera le rendement.

A l'échelle moléculaire, les composés selon l'invention présentent de nombreuses propriétés. La structure même de ces molécules leur confère un système d'électrons π hautement délocalisé sur une longueur relativement grande. Le caractère dissymétrique de cette délocalisation électronique peut être induit par un choix judicieux des substituants X₁ et X₂ ; si X₁ est choisi parmi le groupe de substituants électrodonneurs et X₂ choisi parmi le groupe des substituants électro-accepteurs, la polarité et avant tout la polarisabilité des composés selon l'invention s'en trouvent fortement accrues, et lesdits composés présentent des propriétés optiques non linéaires.

La nature des substituants X₁ et X₂ permet de conférer, aux composés selon l'invention, la réactivité appropriée en vue de leur incorporation au sein de matériaux polymères ou de leur homo- ou co-polymérisation. Enfin, les composés selon l'invention sont mésomorphes et bénéficient d'une haute stabilité thermique.

Selon le choix des substituants X₁ et X₂, les composés de formule générale I présentent une combinaison de propriétés structurales et fonctionnelles qui en font d'excellents candidats comme constituants actifs de dispositifs électroniques, opto-électroniques, optiques non linéaires ou électro-optiques.

Les composés de formule générale I selon l'invention présentent des propriétés intéressantes à l'échelle moléculaire. Il est donc essentiel que les matériaux, dérivant desdits composés et entrant dans l'élaboration des dispositifs précités, conservent ces propriétés à l'échelle macroscopique.

La présente invention concerne des matériaux dérivant des composés selon l'invention. On distinguera quatre classes de matériaux :
. Matériaux moléculaires constitués des composés de formule générale I utilisés comme tels : monocristaux, couches monomoléculaires de Langmuir-Blodgett, sandwich moléculaire, fil moléculaire, composé inclus dans une matrice-hôte organique (polymère thermoplastique par exemple) ou inorganique (verre comme décrit dans EP-A-0 304 051 publié le 22.02.1989, céramique, matrice sol-gel comme décrit dans FR 2 675 509 publié le 23.10.1992) ; dans le cas où les composés selon l'invention sont non réactifs chimiquement.
. Matériaux polymères, en particulier polyoléfine, polyester, polyimide, polyacétate de vinyle, polychlorure de vinyle, polyuréthane, polyméthacrylate de méthyle, hydroxyéthyl-cellulose, sur lesquels sont greffés comme chaînes latérales les composés selon l'invention ; dans le cas où les composés présentent une fonctionnalité supérieure ou égale à 1.

Deux voies de préparation du matériau sont possibles : le composé est soit greffé sur les unités constitutives d'un homopolymère ou d'un copolymère comme décrit dans Polymer, 36(24), 4561 (1995), soit greffé sur un monomère qui subit ensuite une polymérisation ou une copolymérisation.
. Matériaux polymères ou copolymères obtenus par polymérisation ou copolymérisation des composés de formule générale I, dans le cas où lesdits composés ont une fonctionnalité supérieure ou égale à 2.
. Complexes métalliques obtenus par complexation d'une ou plusieurs molécules de formule générale I.

Toute stratégie d'ingéniérie moléculaire permettant d'optimiser les propriétés du matériau à l'échelle microscopique et macroscopique sera élaborée d'abord en choisissant la nature et la réactivité des substituants X₁ et X₂, ensuite en choisissant la mise en forme la plus adaptée à l'application du dispositif visée.

La présente invention concerne l'utilisation des composés et des matériaux selon l'invention en tant que constituants actifs de dispositifs électroniques, opto-électroniques, optiques non linéaires et électro-optiques.

Comme il a été mentionné ci-dessus, les composés selon l'invention présentent un système d'électrons π hautement délocalisé ; ces composés peuvent donc entrer dans l'élaboration de matériaux moléculaires conducteurs ou semi-conducteurs pour des dispositifs purement électroniques, mais également dans l'élaboration de matériaux moléculaires électroluminescents ou photoconducteurs pour des dispositifs opto-électroniques (on entendra par dispositif opto-électronique, tout générateur ou modulateur électrique à commande optique).

Les matériaux selon l'invention, conducteurs ou semi-conducteurs, mis en forme de telle façon qu'ils présentent une caractéristique courant-tension non linéaire, éventuellement associée à des effets redresseurs et/ou de mémoire, sont intéressants comme composants actifs dans la réalisation de dispositifs électroniques, par exemple, les diodes et les transistors.

Les matériaux selon l'invention électroluminescents peuvent entrer dans l'élaboration de diodes électroluminescentes (LED) utiles pour les dispositifs d'affichage actifs, comme décrit dans J.H. Burroughes et al., "Nature" 347, 1990, page 539. En particulier, le composé de formule générale I greffé comme chaîne latérale sur un polyester, permet l'obtention d'un polymère électroluminescent dont la mise en forme est aisée pour l'élaboration d'une LED.

Les matériaux selon l'invention photoconducteurs, par exemple le composé de formule générale I polymérisé ou greffé comme chaîne latérale sur un polymère, permettent l'enregistrement d'images électrostatiques utile en reproduction d'images, par exemple la xérographie, la photocopie ou l'impression laser. Le brevet FR 2 712 893 publié le 02.06.1995 décrit un film de polymère de silane greffé permettant de telles applications. Lesdits matériaux photoconducteurs associés en série à un film de cristal liquide, aux bornes desquels on applique une tension, définissent un modulateur spatial de lumière adressé optiquement capable de transformer une lumière incohérente en lumière cohérente, ou de transformer un rayonnement infrarouge en lumière visible et inversement.

Comme il a été mentionné ci-dessus, les composés selon l'invention présentent un système d'électrons π délocalisé sur l'ensemble de la molécule conjuguée. Cette délocalisation peut être renforcée par l'action coopérative d'un groupement électrodonneur, par exemple X₁, et d'un groupement électro-accepteur, par exemple X₂, placés chacun en bout de chaîne. Le nuage électronique ainsi polarisé interagit avec un champ électromagnétique de pulsation ω_{O} pour induire une réponse optique non linéaire, par exemple un champ électromagnétique de pulsation ω ≠ ω_{O}. De tels composés permettent la mise en oeuvre de dispositifs optiques non linéaires, sachant qu'un bon matériau pour de tels dispositifs devra non seulement intégrer lesdits composés selon l'invention non centrosysmétriques mais aussi les intégrer de façon non centrosymétrique : le composé de formule générale I, telle que X₁ = NH₂ et X₂ = NO₂, greffé sur une polyimide fournit un matériau répondant à ces exigences.

Les interactions des matériaux et composés selon l'invention avec le ou les pulsations incidentes peuvent être de différents types : doublage de fréquence incidente (application aux dispositifs de mémoires optiques à haute densité), addition ou différence de deux fréquences incidentes (application aux convertisseurs infrarouge- visible et aux redresseurs optiques), émission ou amplification paramétrique (application aux sources cohérentes accordables et au laser paramétrique). Ces différents processus permettent d'engendrer de nouvelles fréquences à partir de la ou des fréquences incidentes sur un domaine spectrale situé du proche UV au proche IR. Si en outre le matériau optique non linéaire selon l'invention est soumis à un champ électrostatique ou électromagnétique de basse fréquence, l'interaction pulsation incidente/matière aura pour effet de modifier non seulement la fréquence incidente de l'onde incidente mais aussi son amplitude, sa phase et son trajet : les composés selon l'invention optiques non linéaires peuvent entrer dans l'élaboration de dispositifs électro-optiques (on entendra par dispositif électro-optique tout générateur ou modulateur optique à commande électrique), notamment les modulateurs et commutateurs électro-optiques utiles pour le traitement du signal optique et les télécommunications optiques.

Enfin, comme il a été mentionné ci-dessus, les composés selon l'invention présentent un caractère mésomorphe qui permet d'envisager leur application dans des dispositifs électro-optiques d'affichage à cristaux liquides passif.

Les exemples suivants illustreront l'invention sans en restreindre la portée.

### Exemple 1: Préparation de la 6,6'-distyryl-3,3'-bipyridine

Dans un ballon bicol de 50 ml sont successivement introduits 2,7 g de 6,6'-méthyl-3,3'-bipyridine (DMBP), soit 1,5.10⁻² mole, 6,5 g (6,3.10⁻² mole) de benzaldéhyde, et 7,9 g (3,5.10⁻² mole) d'anhydride benzoïque servant à la fois de catalyseur et d'agent déshydratant. Le mélange, chauffé au bain d'huile à 200°C, est laissé à reflux sous léger courant de gaz inerte pendant 1,5 heure, en suivant l'avancement de la réaction par chromatographie d'exclusion. Au bout du temps indiqué, la DMBP a complètement disparu et le mélange n'évolue plus. Le mélange réactionnel est lavé à l'éthanol et à l'éther. Le résidu de couleur brun sombre est recristallisé dans le toluène ; on obtient ainsi 1,7 g d'un produit encore brunâtre. Un produit pur à 98%, est obtenu par nouvelle cristallisation dans le toluène en présence de charbon actif. Quantité obtenue : 1,43 g (0,4.10⁻² mole) ; rendement : 27 %).

Le composé obtenu, qui est un corps cristallisé sous la forme de paillettes de couleur jaune clair d'apparence légèrement fluorescente, possède la particularité de donner lieu au-dessus de 238°C à la formation d'une phase cristal liquide de type smectique A, se transformant elle-même à partir de 280°C en phase nématique, elle-même fondant à 311°C. En solution, ce composé est effectivement fluorescent, avec un maximum d'émission à 424 nm, alors que le pic d'absorption se situe à 350 nm.

### Exemple 2 : Préparation de la 6,6'-di(4-cyano-styryl)-3,3'-bipyridine

On utilise les mêmes conditions opératoires que dans l'exemple 1.

Le mélange, composé de 1,18 g de 6,6'-diméthyl-3,3'-bipyridine (DMBP) soit 6,4.10⁻³ mole, 3,57 g de 4-cyanobenzaldéhyde (27,2.10⁻³ mole) et 3,43 g d'anhydride benzoïque, est chauffé au bain d'huile à 180°C pendant 3 heures. Le mélange réactionnel, une fois revenu à température ambiante, est lavé à l'éthanol puis à l'éther. Le résidu (1,92 g) est recristallisé dans 95 ml de diméthylformamide. Un produit pur à 97 % est obtenu sous forme de cristaux jaune se transformant à 305°C en phase cristal liquide.Quantité obtenue : 1,59 g soit 3,9.10⁻³ mole ; rendement : 61 %.

### Exemple 3 : Préparation de la 6,6'-di(4-hydroxy-styryl)-3,3'-bipyridine

On utilise les mêmes conditions opératoires que dans l'exemple 1.

Le mélange, composé de 943,1 mg de 6,6'-diméthyl-3,3'-bipyridine (DMBP) soit 5,12.10⁻³ mole, 2,75 g de 4-hydroxybenzaldéhyde (22,5.10⁻³ mole) et 2,75 g d'anhydride benzoïque, est chauffé au bain d'huile à 180°C pendant 4 heures. Le mélange réactionnel, une fois revenu à température ambiante, est lavé à l'éthanol puis à l'éther. Après saponification dans une solution alcoolique de soude 2N pendant 15 minutes à 80°C et précipitation dans l'eau, un précipité (1,01 g) est recristallisé dans 75 ml de diméthylformamide. Un produit pur à 98 % est obtenu, présentant une transformation en phase cristal liquide. Quantité obtenue: 942 mg soit 2,4.10⁻³ mole ; rendement : 47 %.

### Exemple 4 : Préparation de la 6-(4-hydroxy-styryl)-6'-(4-cyano-styryl)-3,3'-bipyridine.

La première étape de la synthèse est effectuée dans les mêmes conditions dans l'exemple 1 avec 799,6 mg de 6,6'-diméthyl-3,3'-bipyridine (DMBP) soit 4,3.10⁻³ mole, 569,0 mg de 4-cyanobenzaldéhyde (4,3.10⁻³ mole) et 1,5 g d'anhydride benzoïque (6,6.10⁻³ mole). Le mélange est chauffé au bain d'huile à 140°C sous léger courant de gaz inerte pendant 3 heures. Le mélange réactionnel, une fois revenu à température ambiante, est lavé à l'éthanol puis à l'éther. Au résidu sont additionnés successivement 530 mg de 4-hydroxybenzaldéhyde (4,3.10⁻³ mole) et 1,21 g d'anhydride benzoïque (5,3.10⁻³ mole). Le mélange est chauffé au bain d'huile à 160°C sous léger courant de gaz inerte pendant 10 heures. Le mélange réactionnel, une fois revenu à température ambiante, est lavé à l'éthanol puis à l'éther. Après saponification dans une solution alcoolique de soude 2N pendant 15 minutes à 80°C et précipitation dans l'eau, un précité pur à 82 % est obtenu. Quantité obtenue : 351,7 mg soit 9.10⁻⁴ mole ; rendement : 21 %.

## Revendications

1. Composés de formule générale I 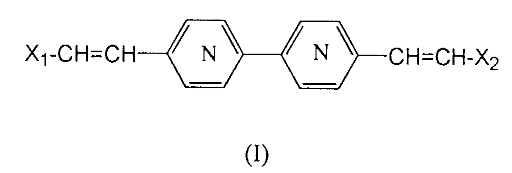 dans laquelle
l'entité 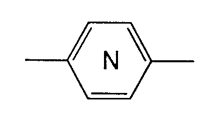 représente un radical hétérocyclyle aromatique azoté comprenant 1 à 4 atomes d'azote, choisi parmi pyridinyle, pyrazinyle, pyrimidinyle, triazinyle, et comportant au moins un atome d'azote en position α par rapport à la double liaison CH=CH,
X₁ et X₂ sont indépendamment l'un de l'autre des systèmes conjugués aliphatiques eux-mêmes conjugués avec le reste de molécule, cycliques aromatiques ou hétérocycliques aromatiques, en particulier un phényle ou un thiényle, non substitués ou substitués une ou plusieurs fois par des radicaux choisis parmi le groupe alkyle, halogéno, phényle, naphtyle, hydroxyle, alkoxy, amino, alkyl inférieur-amino, dialkyl inférieur-amino, alkyl inférieur-alkoxy inférieur-amino, (alkyl inférieur-acyl)-amino, carbonyle, alkyl inférieur-carbonyle, alkoxy inférieur-carbonyle, carboxyle, (alkyl inférieur-amino)-carbonyle, (dialkyl inférieur-amino)-carbonyle, halogéno-carbonyle, trifluoro-méthyle, nitro, nitroso, cyano, 2,2-dicyano-vinyle,3,3-dicyano-prop-2-ényle,tricyano-vinyle, dicyanométhylidényle, mercapto, alkylthio, sulfino, alkyl-sulfonyle, sulfo, alkyl-sulfonate, alkyl-sulfinyle, pyridinyle et dérivés de pyridinyle conjugués.

2. Composés selon la revendication 1, caractérisés en ce que l'entité 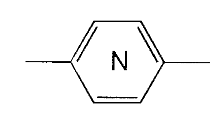 représente un radical pyridinyle comportant un atome d'azote en position α par rapport à la double liaison CH=CH.

3. Composés selon la revendication 2, caractérisés en ce que X₁ et X₂ représentent indépendamment l'un de l'autre un phényle, un 4-cyano-phényle ou un 4-hydroxy-phényle.

4. Composés selon la revendication 3, caractérisés en ce qu'ils sont choisis parmi la 6,6'-distyryl-3,3'-bipyridine, la 6,6'-di(4-cyano-styryl)-3,3'-bipyridine, la 6,6'-di(4-hydroxy-styryl)-3,3'-bipyridine et la 6-(4-hydroxy-styryl)-6'-(4-cyano-styryl)-3,3'-bipyridine.

5. Procédé de préparation des composés selon la revendication 1 de formule générale I dans laquelle X₁ = X₂, caractérisé en ce que l'on condense le produit de formule générale II 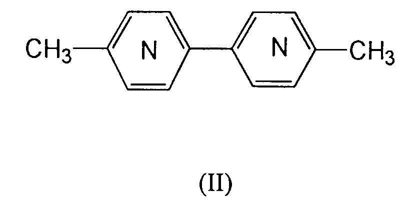 dans laquelle l'entité 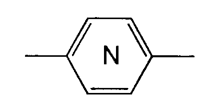 représente un radical hétérocyclyle aromatique azoté comprenant 1 à 4 atomes d'azote, choisi parmi pyridinyle, pyrazinyle, pyrimidinyle, triazinyle, et comportant au moins un atome d'azote en position α par rapport au radical méthyle,
avec un excès d'aldéhyde de formule X-CHO
avec X=X₁=X₂, X₁ et X₂ étant définis comme précédemment.

6. Procédé de préparation des composés selon la revendication 1 de formule générale I dans laquelle X₁ ≠ X₂, caractérisé en ce que l'on condense le produit de formule générale II dans laquelle chaque radical hétérocyclyle aromatique azoté est défini comme précédemment, successivement avec un équivalent molaire d'aldéhyde de formule X₁-CHO, dans laquelle X₁ est défini ci-dessus, puis avec un équivalent molaire d'aldéhyde X₂-CHO, dans laquelle X₂ ≠ X₁ et X₂ est défini ci-dessus.

7. Procédé selon la revendication 5 ou 6, caractérisé en ce que la condensation a lieu en présence d'un catalyseur et d'un agent déshydratant.

8. Matériaux dérivant des composés selon la revendication 1, de préférence polymériques.

9. Utilisation des composés selon la revendication 1 et des matériaux selon la revendication 8, en tant que constituants actifs de dispositifs électroniques, par exemple diode ou transistor, opto-électroniques, par exemple LED ou modulateur spatial de lumière, optiques-non-linéaires, par exemple doubleur de fréquence, laser paramétrique ou convertisseur infrarouge-visible, et électro-optiques, par exemple modulateur, commutateur ou afficheur à cristaux liquides.
